# EUROPEAN PATENT SPECIFICATION

(11) **EP 3 884 842 B1**
(45) Date of publication and mention of the grant of the patent: **26.02.2025**
(21) Application number: 20818131.3
(22) Date of filing: 04.06.2020
(51) Int. Cl.: A61B 1/00, A61B 1/018, A61B 1/307, A61B 17/32, A61B 17/3209, A61B 18/00, A61B 18/14

(54) **MULTIFUNCTIONAL ENDOSCOPIC SYSTEM FOR CAVITY INSPECTION AND TREATMENT**
MULTIFUNKTIONELLES ENDOSKOPISCHES SYSTEM ZUR INSPEKTION UND BEHANDLUNG VON HOHLRÄUMEN
SYSTÈME ENDOSCOPIQUE MULTIFONCTIONNEL DESTINÉ À L'INSPECTION ET AU TRAITEMENT DE CAVITÉS

(30) Priority: 04.06.2019 CN 201910479922
(43) Date of publication of application: 29.09.2021
(73) Proprietor: Peking University First Hospital, Beijing 100034 (CN)
(72) Inventor: LI, Xuesong, Beijing 100034 (CN); ZHOU, Liqun, Beijing 100034 (CN); GUO, Yinglu, Beijing 100034 (CN); BAO, Guohua, Beijing 100034 (CN); ZHOU, Yan, Beijing 100034 (CN); MA, Jianqiang, Beijing 100034 (CN); DING, Guangpu, Beijing 100034 (CN); GONG, Kan, Beijing 100034 (CN); WANG, Gang, Beijing 100034 (CN); YANG, Kunlin, Beijing 100034 (CN); XIONG, Gengyan, Beijing 100034 (CN); HONG, Peng, Beijing 100034 (CN); FAN, Shubo, Beijing 100034 (CN)
(74) Representative: Groth & Co. KB
(86) International application number: PCT/CN2020/094464
(87) International publication number: WO 2020/244602

(56) References cited:
- CN-A- 110 123 255
- CN-U- 201 519 129
- CN-U- 210 354 645
- CN-Y- 2 855 340
- CN-Y- 201 179 104
- DE-A1- 102016 010 548
- DE-A1- 102017 114 449
- GB-A- 2 545 559
- JP-A- H09 262 245
- US-A- 5 041 111
- US-A- 5 112 330
- US-A- 5 925 040
- US-A1- 2004 242 959
- US-A1- 2015 351 826
- US-A1- 2015 351 826
- US-A1- 2019 069 946

## Description

### TECHNICAL FIELD

The present invention relates to a multifunctional endoscopic system for cavity inspection and treatment, in particular to a multifunctional endoscopic system for cavity inspection and treatment applied to the diagnosis and treatment of the ureteral cavity.

### BACKGROUD

Ureteral stricture and obstruction are common causes of hydronephrosis. Many types of diseases, comprising benign and malignant diseases, may cause ureteral stricture. Ureteroscopy is an important way to diagnose and treat ureteral obstruction. According to the structural characteristics of ureteroscopy, it can be classified into flexible ureteroscope, rigid ureteroscope, and semi-rigid ureteroscope and so on. The existing ureteroscope has a single function, and different ureteroscopes need to be replaced for different treatment operations, which brings inconvenience to the operation of medical staff.

Therefore, there is an urgent need to develop a multifunctional endoscopic system for cavity inspection and treatment on ureter, including endoureterotomy, monopolar electric resection, bipolar electric resection, laser resection, catheter placement, and clamping.

Document US5112330A describes a resectoscope apparatus for such treatments as resecting and coagulating tissues within a body cavity according to the preamble of claim 1.

### SUMMARY

In view of the above problems, the present application is intended to provide a multifunctional endoscopic system for cavity inspection and treatment.

In order to achieve the above objective, the present application adopts the following technical solutions, a multifunctional endoscopic system as defined in claim 1.

Preferably, the internal incision piece comprise an internal incision knife, and is formed with a blade portion at a front end thereof, and a snap-fitting portion at a rear end thereof; and a plurality of cylindrical connecting pieces are spaced apart on an outer side wall of an middle portion of the internal incision piece, and the connecting piece are open at both ends thereof.

Preferably, the internal incision knife is a long handled cold knife; and the internal incision knife has a knife head, and the knife head is straight, semicircular or hook.

Preferably, the internal incision piece comprises an internal incision electrode, and is formed with an electrode portion at a front end of the internal incision piece, and a snap-fitting portion at a rear end of the internal incision piece; and a cylindrical connecting piece is arranged on an outer side wall of an middle portion of the internal incision piece, and the connecting piece is open at both ends thereof.

Preferably, the electrode portion of the internal incision electrode is a needle electrode or a ring electrode.

Preferably, the endoscope adopts a straight rigid tube fiber endoscope with a diameter less than ϕ2 mm; and the endoscope sheath has a diameter less than ϕ2.5 mm.

The present application adopting the above technical solution, has the following advantages: The present application provides a multifunctional endoscopic system for cavity inspection and treatment, which includes an internal incision working hand piece, and an internal incision piece arranged on the internal incision working hand piece, a endoscope sheath connected to a front end of the internal incision working hand piece, and an endoscope connected to a back end of the internal incision working hand piece. The internal incision piece adopts an internal incision knife or an internal incision electrode, and the corresponding internal incision working hand piece adopts cold knife type internal incision working hand piece or monopolar and bipolar mixed-type internal incision working hand piece. According to the treatment requirements, different instruments can be chosen and combined for treatment, and to perform ureteroscopy to the ureter, and perform multiple treatment operations comprising internal incision, monopolar electric resection, bipolar electric resection, laser resection, etc., which are convenient for medical staff to perform operation treatment. The endoscopic system of the present application has an elongate endoscope sheath, precise orientation, and complete functions, and it is easier for the endoscopic system to enter a narrow segment of the cavity for diagnosis, and different treatment methods can be selected according to treatment requirements.

### BRIEF DESCRIPTION OF THE DRAWINGS

Figure 1 is a schematic view of the overall structure of Example1 of the present application.
Figure 2 is a schematic structural view of the endoscope bridge in Example1 of the present application.
Figure 3 is a schematic structural front view of the internal incision knife in Example 1 of the present application.
Figure 4 is a schematic structural top view of the internal incision knife in Example 1 of the present application.
Figure 5 is a schematic structural view of the mono-polar and bipolar mixed-type internal incision working hand piece according to Example 2 of the present application. Figure
Figure 6 is a schematic structural view of the needle electrode in Example 2 of the present application.
Figure 7 is a schematic structural front view of the ring electrode in Example 2 of the present application.
Figure 8 is a schematic structural top view of the ring electrode in Example2 of the present application.

### DETAILED DESCRIPTION

The present invention will be described in detail below with reference to the appended drawings and embodiments. Apparently, it should be understood that the drawings are only provided to better understand the present application, but not to limit the present application.

### EXAMPLE 1

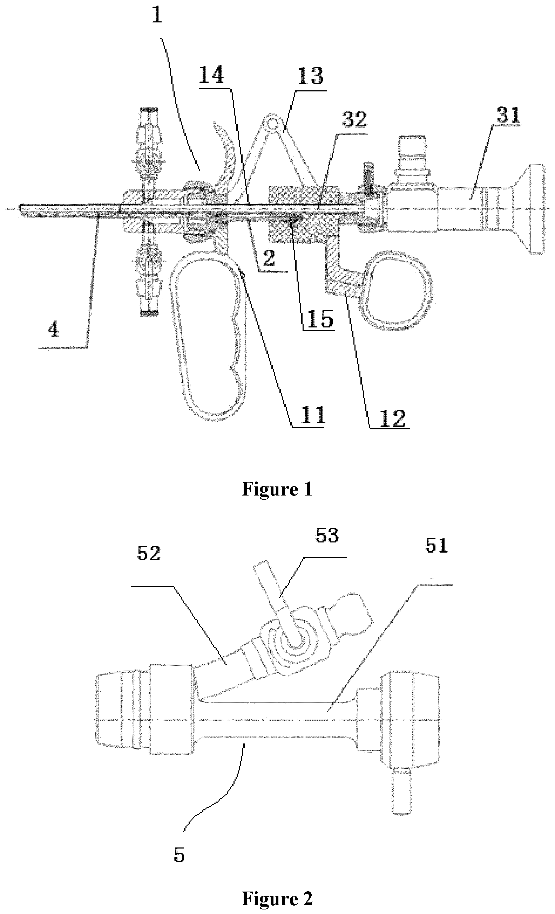

As shown in Figure 1, a multifunctional endoscopic system for cavity inspection and treatment is provided in this embodiment, it comprises
an internal incision working hand piece 1;
an endoscope 3, arranged to insert into the internal incision working hand piece 1 from a rear end of the internal incision working hand piece 1, wherein a head part 31 of the endoscope 3 is snap-fitted on the rear end of the internal incision working hand piece 1, and an extension part 32 of the endoscope 3 passes through an inner cavity of the internal incision working hand piece 1 and extends from a front end of the internal incision working hand piece 1;
an internal incision knife 2, located below the extension part 32 of the endoscope 3 and distributed along an axial direction of the extension part 32, wherein the rear end of the internal incision knife 2 is arranged to snap-fit fixedly on the internal incision working hand piece 1;
an endoscope sheath 4, snap-fitted on the front end of the internal incision working hand piece 1, wherein the internal incision piece 2 and the extension part 32 of the endoscope 3 are axially sleeved in the endoscope sheath 4.

Further, as shown in Figure 2, this embodiment also comprises an endoscope bridge 5, which comprises a main channel 51 used to replace the internal incision working hand piece 1 and connected with the endoscope 3 and the endoscope sheath 4; and a bypass channel 52 connected with the main channel 51, the bypass channel 52 is provided with an on-off valve 53. The bypass channel 52 is opened when performing treatment operations such as laser resection, catheter placement, and clamping.

Further, the internal incision working hand piece 1 adopts a cold knife type internal incision working hand piece, comprising a fixed hand piece 11; an operation handle 12, arranged on the fixed hand piece 11 and used to push the internal incision piece 2 to move forward and back axially along the extension part 32 of the endoscope 3, and a V-shape resilient piece 13 arranged between the fixed hand piece 11 and the operation handle 12 for resetting the operation handle 12. A channel 14 is formed between the fixed hand piece 11 and the operation handle 12 for the extension part 32 of the endoscope 3 to pass through. A mounting slot 15 is formed between the fixing hand piece 11 and the operation handle 12 for fixing the internal incision piece 2.

Further, a limiting block (not shown in the figure) is arranged on a side of the operation handle 12 close to the fixed hand piece 11 and protruded therefrom. During operation, the incision knife 2 is pushed by the operator through the operation handle 12 to move and extend to the outside of the endoscope sheath 4, when the limiting block is against a side wall of the fixed hand piece 11, the continuous extension of the internal incision knife 2 is stopped, and the operator is informed that the internal incision knife 2 has reached a specified length, which is convenient for the operator to operate and improve operating efficiency. Preferably, a groove (not shown in the figure) for receiving the limiting block is arranged on the operation handle 12, and the limiting block is rotatably arranged in the groove.

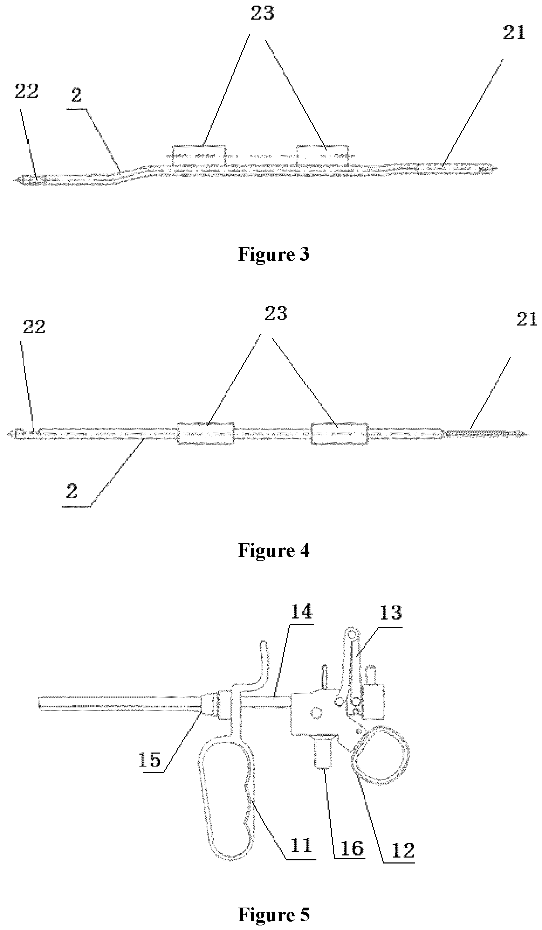

Further, as shown in Figures 3 and 4, the internal incision knife 2 is formed with a blade portion 21 at a front end thereof, and a snap-fitting portion 22 at a rear end thereof; and a plurality of cylindrical connecting pieces 23 are spaced apart on an outer side wall of an middle portion of the internal incision piece 2, and the connecting piece 23 are open at both ends thereof. The extension part 32 of the endoscope 3 passes through the plurality of connecting pieces 23 in sequence, such that the internal incision knife 2 is axially distributed below the extension part 32 of the endoscope 3, the rear end of the internal incision knife 2 is inserted into the internal incision working hand piece 1, and the internal incision knife 2 is arranged to snap-fit fixedly on the internal incision working hand piece 1 with the snap-fitting portion 22 on the internal incision knife 2. When the internal incision working hand piece 1 is used to push the internal incision knife 2 to move along the axial direction of the extension part 32 of the endoscope 3, the connecting piece 23 plays a guiding role.

Further, the internal incision knife 2 is a long handled cold knife; and the internal incision knife has a knife head, and the knife head is straight, semicircular or hook.

Further, the endoscope 3 adopts a straight rigid tube fiber endoscope with a diameter less than ϕ2 mm; and the endoscope sheath 4 has a diameter less than ϕ2.5 mm.

The usage method of this embodiment is as follows:
Taking the endoscopic system for inspection and treatment in this embodiment to treat middle and upper ureteral stricture through a urethra as an example, a normal saline perfusion is used during the operation.
① Catheterization: connecting the endoscope 3 with the endoscope sheath 4 and the endoscope bridge 5, and connecting the endoscope 3 with a light source and camera system, and a perfusion pump, and starting the operation; entering the bladder through the urethra;
   inserting a guide wire through the endoscope bridge 5, and bring the endoscope into the ureter under the guidance of the guide wire to reach a stricture part, forwarding the guide wire, and if passing through the stricture part retrogradely, fixing the guide wire and withdrawing the endoscope.
② Disassembly and connection: disassembling the endoscope 3, the endoscope sheath 4, and the endoscope bridge 5, connecting the endoscope 3 with the internal incision working hand piece 1, and mounting the internal incision knife 2 into the internal incision working hand piece 1 and fixing it with hand piece latch, sleeving the endoscope 3 and the internal incision knife 2 into the endoscope sheath 4, and connecting the endoscope sheath 4 to the front end of the internal incision working hand piece 1.
③ Cold knife internal incision under the endoscope: forwarding the assembled endoscopic system for inspection and treatment to the stricture part along the guide wire under the guidance of the guide wire, controlling the operation of internal incision working hand piece 1 such that the internal incision knife 2 is extended to the outside of the scope of the sheath 4, after incising the stricture part according to therapy principle, retracting the internal incision knife 2 into the endoscope sheath 4, and forwarding the endoscope through the stricture part, and after endoscope confirmation, fixing the guide wire and withdrawing the endoscope;inserting a double J tube through the guide wire, withdrawing the guide wire under the endoscope, observing the locating position of the double J tube, and after the confirmation, withdrawing the endoscope and ending the surgery; during the above operation, a C-arm X-ray machine is used to monitor and guide as needed.

### EXAMPLE 2

As shown in Figures 5 to 8, compared with Example 1, a multifunctional endoscopic system for cavity inspection and treatment is provided in this embodiment, it comprises
a monopolar and bipolar mixed type internal incision working hand piece 1,
an internal incision electrode 2, arranged to snap-fit fixedly on the monopolar and bipolar mixed type internal incision working hand piece 1,
an endoscope 3, arranged to insert into the monopolar and bipolar mixed type internal incision working hand piece 1 from a rear end of monopolar and bipolar mixed type internal incision working hand piece 1, wherein a head part 31 of the endoscope 3 is snap-fitted on the rear end of the monopolar and bipolar mixed type internal incision working hand piece 1, and an extension part 32 of the endoscope 3 passes through an inner cavity of the monopolar and bipolar mixed type internal incision working hand piece 1 and extends from a front end of the monopolar and bipolar mixed type internal incision working hand piece 1;
an endoscope sheath 4, snap-fitted on the front end of the monopolar and bipolar mixed type internal incision working hand piece 1, wherein the internal incision electrode 2 and the extension part 32 of the endoscope 3 are axially sleeved in the endoscope sheath 4.

Further, the monopolar and bipolar mixed type internal incision working hand piece 1 comprises a fixed hand piece 11; an operation handle 12, arranged on the fixed hand piece 11 and used to push the internal incision electrode 2 to move forward and back axially along the extension part 32 of the endoscope 3, and a V-shape resilient piece 13 arranged between the fixed hand piece 11 and the operation handle 12 for resetting the operation handle 12. A channel 14 is formed between the fixed hand piece 11 and the operation handle 12 for the extension part 32 of the endoscope 3 to pass through. A mounting slot 15 is formed between the fixing hand piece 11 and the operation handle 12 for fixing the internal incision electrode 2; and the operation handle 12 is provided with an electrode connecting socket for connecting an electrode.

Further, the electrode connection socket 16 comprises a monopolar socket and a bipolar socket arranged on the operation handle 12.

Further, the internal incision electrode 2 is formed with an electrode portion 24 at a front end thereof, and a snap-fitting portion 25 at a rear end thereof; and a plurality of cylindrical connecting pieces 26 are spaced apart on an outer side wall of an middle portion of the internal incision electrode 2, and the connecting piece 26 are open at both ends thereof. The extension part 32 of the endoscope 3 passes through the plurality of connecting pieces 26 in sequence, such that the internal incision electrode 2 is axially distributed below the extension part 32 of the endoscope 3, the rear end of the internal incision electrode 2 is inserted into the monopolar and bipolar mixed type internal incision working hand piece 1, and the internal incision electrode 2 is arranged to snap-fit fixedly on the monopolar and bipolar mixed type internal incision working hand piece 1 with the snap-fitting portion 25 on the internal incision electrode 2. When the mono-polar and bipolar mixed type internal incision working hand piece 1 is used to push the internal incision electrode 2 to move along the axial direction of the extension part 32 of the endoscope 3, the connecting piece 26 plays a guiding role.

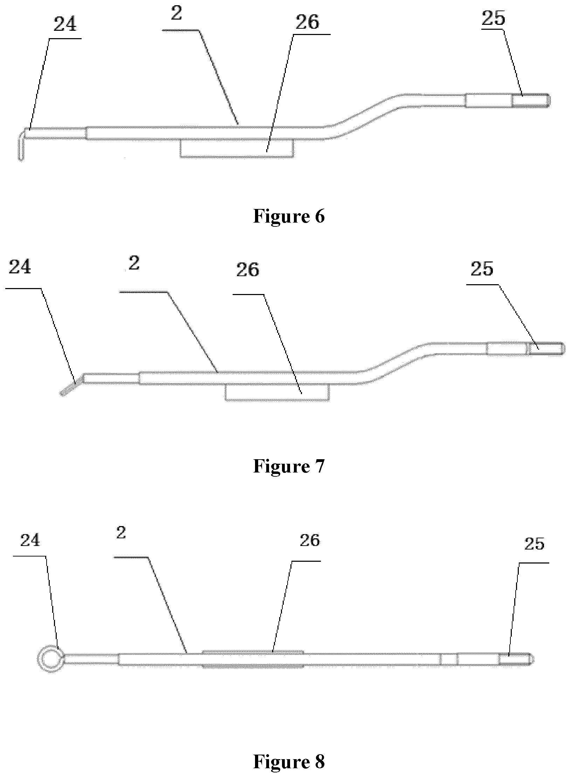

Further, as shown in Figure 6 to Figure 8, the electrode portion 24 located at the front end of the internal incision electrode 2 adopts a needle electrode or a ring electrode.

Further, this embodiment also comprises an endoscope bridge 5, which comprises a main channel 51 used to replace the monopolar and bipolar mixed type internal incision working hand piece 1 and connected with the endoscope 3 and the endoscope sheath 4; and a bypass channel 52 that is connected with the main channel 51, the bypass channel 52 is provided with an on-off valve 53. The bypass channel 52 is opened when performing treatment operations such as laser resection, catheter placement, and clamping.

The usage method of this embodiment is as follows:
Taking the endoscopic system for inspection and treatment in this embodiment to treat middle and upper ureteral stricture through a urethra as an example, a solution perfusion of the solution having 4% by volume of mannitol or 5% by volume of glucose is used during the surgery operation.
① Catheterization: connecting the endoscope 3 with the endoscope sheath 4 and the endoscope bridge 5, and connecting the endoscope 3 with a light source and camera system, and a perfusion pump, and starting the operation; entering the bladder through the urethra;
   inserting a guide wire through the endoscope bridge 5, and bring the endoscope into the ureter under the guidance of the guide wire to reach a stricture part, forwarding the guide wire, and if passing through the narrow part retrogradely, fixing the guide wire and withdrawing the endoscope.
② Disassembly and connection: disassembling the endoscope 3, the endoscope sheath 4, and the endoscope bridge 5, connecting the endoscope 3 with the monopolar and bipolar mixed type internal incision working hand piece 1, and mounting the internal incision electrode 2 into the mono-polar and bipolar mixed type internal incision working hand piece 1 and fixing it with a hand piece latch, sleeving the endoscope 3 and the internal incision electrode 2 into the endoscope sheath 4, and connecting the endoscope sheath 4 to the front end of the monopolar and bipolar mixed type internal incision working hand piece 1;
   plugging one end of the monopolar and bipolar lead wire into the monopolar socket of the monopolar and bipolar mixed-type internal incision working hand piece1, and connecting the other end of the monopolar and bipolar lead wire to the monopolar output port of an energy platform.
③ Monopolar electric resection type internal incision under the endoscope: forwarding the assembled endoscopic system for inspection and treatment to the stricture part along the guide wire under the guidance of the guide wire, controlling the operation of the monopolar and bipolar mixed type internal incision working hand piece 1 such that the internal incision electrode 2 is extended to the outside of the scope of the sheath 4, after incising the stricture part according to therapy principle, retracting the internal incision electrode 2 into the endoscope sheath 4, and forwarding the endoscope through the stricture part, and after endoscope confirmation, fixing the guide wire and withdrawing the endoscope; inserting a double J tube through the guide wire, withdrawing the guide wire under the endoscope, observing the locating position of the double J tube, and after the confirmation, withdrawing the endoscope and ending the surgery; during the above operation, a C-arm X-ray machine is used to monitor and guide as needed.

In addition, another usage method of this embodiment is as follows
Compared with the above-mentioned usage method, other processes remain unchanged, and the only difference lies in that only one end of the monopolar and bipolar lead wire is plugged into the bipolar socket of the monopolar and bipolar mixed-type internal incision working hand piece 1, and the other end of the monopolar and bipolar lead wire is connected to the bipolar output port of the energy platform, such that the bipolar electric resection type internal incision under the endoscope can be performed.

## Claims

1. A multifunctional endoscopic system for cavity inspection and treatment, comprising
an internal incision working hand piece (1);
an internal incision piece (2), arranged to snap-fit fixedly on the internal incision working hand piece (1),
an endoscope (3), arranged to insert into the internal incision working hand piece (1) from a rear end of the internal incision working hand piece (1), wherein a head part (31) of the endoscope (3) is snap-fitted on the rear end of the internal incision working hand piece (1), and an extension part (32) of the endoscope (3) passes through an inner cavity of the internal incision working hand piece (1) and extends from a front end of the internal incision working hand piece (1);
an endoscope sheath (4), snap-fitted on the front end of the internal incision working hand piece (1), wherein the internal incision piece (2) and the extension part (32) of the endoscope (3) are axially sleeved in the endoscope sheath (4),
wherein the internal incision working hand piece (1) comprises:
a fixed hand piece (11);
an operation handle (12), arranged on the fixed hand piece (11) and used to push the internal incision piece (2) to move forward and back axially along the extension part (32) of the endoscope (3), and
a V-shape resilient piece (13) for resetting the operation handle (12); wherein,
a channel (14) is formed between the fixed hand piece (11) and the operation handle (12) for the extension part (32) of the endoscope (3) to pass through,
**characterized in that** a mounting slot (15) is formed between the fixing hand piece (11) and the operation handle (12) for fixing the internal incision piece (2), and
a limiting block is arranged on a side of the operation handle (12) close to the fixed hand piece (11) and protruded therefrom, so as to be against a side wall of the fixed hand piece (11) during operation, and a groove for receiving the limiting block is arranged on the operation handle (12), and the limiting block is rotatably arranged in the groove.

2. The multifunctional endoscopic system for cavity inspection and treatment of claim 1, **characterized in that**
the internal incision working hand piece (1) adopts a cold knife type internal incision working hand piece.

3. The multifunctional endoscopic system for cavity inspection and treatment of claim 1, **characterized in that**
the internal incision working hand piece (1) adopts a mono-polar and bipolar mixed-type internal incision working hand piece,
and an electrode connecting socket (16) is arranged on the operation handle (12).

4. The multifunctional endoscopic system for cavity inspection and treatment of claim 1, **characterized in that**
the internal incision piece (2) comprises an internal incision knife, and is formed with a blade portion (21) at a front end thereof, and a snap-fitting portion (22) at a rear end thereof; and
a plurality of cylindrical connecting pieces (23) are spaced apart on an outer side wall of an middle portion of the internal incision piece (2), and the connecting piece (23) are open at both ends thereof.

5. The multifunctional endoscopic system for cavity inspection and treatment of claim 4, **characterized in that**
the internal incision knife is a long handled cold knife; and the internal incision knife has a knife head, and the knife head is straight, semicircular or hook.

6. The multifunctional endoscopic system for cavity inspection and treatment of claim 1, **characterized in that**
the internal incision piece (2) comprises an internal incision electrode, and is formed with an electrode portion (24) at a front end of the internal incision piece (2), and a snap-fitting portion (25) at a rear end of the internal incision piece (2); and
a cylindrical connecting piece (26) is arranged on an outer side wall of a middle portion of the internal incision piece (2), and the connecting piece (23) is open at both ends thereof.

7. The multifunctional endoscopic system for cavity inspection and treatment of claim 6, **characterized in that**
the electrode portion (24) of the internal incision electrode is a needle electrode or a ring electrode.

8. The multifunctional endoscopic system for cavity inspection and treatment of claim 1, **characterized in that**
the endoscope (3) adopts a straight rigid tube fiber endoscope with a diameter less than ϕ2 mm; and the endoscope sheath (4) has a diameter less than ϕ2.5 mm.

## Patentansprüche

1. Multifunktionales endoskopisches System zur Hohlraumuntersuchung und - behandlung, umfassend
ein Arbeitshandstück zur internen Inzision (1);
ein Stück zur internen Inzision (2), das angeordnet ist, um fest am Arbeitshandstück zur internen Inzision (1) einzuschnappen,
ein Endoskop (3), das angeordnet ist, um von einem hinteren Ende des Arbeitshandstück zur internen Inzision (1) in das Arbeitshandstück zur internen Inzision (1) eingesetzt zu werden, wobei ein Kopfteil (31) des Endoskops (3) am hinteren Ende des Arbeitshandstücks zur internen Inzision (1) eingeschnappt ist und ein Erweiterungsteil (32) des Endoskops (3) durch einen inneren Hohlraum des Arbeitshandstücks zur internen Inzision (1) hindurchgeht und sich aus einem vorderen Ende des Arbeitshandstücks zur internen Inzision (1) erstreckt;
eine Endoskophülle (4), die am vorderen Ende des Arbeitshandstücks zur internen Inzision (1) eingeschnappt ist, wobei das Stück zur internen Inzision (2) und das Erweiterungsteil (32) des Endoskops (3) axial in der Endoskophülle (4) eingehüllt sind,
wobei das Arbeitshandstück zur internen Inzision (1) umfasst:
ein feststehendes Handstück (11);
einen Betätigungsgriff (12), der an dem feststehenden Handstück (11) angeordnet ist und verwendet wird, um das Stück zur internen Inzision (2) zu schieben, um es axial entlang des Erweiterungsteils (32) des Endoskops (3) zu bewegen, und
ein V-förmiges elastisches Stück (13) zum Zurücksetzen des Betätigungsgriffes (12); wobei
ein Kanal (14) zwischen dem feststehenden Handstück (11) und dem Betätigungsgriff (12) gebildet ist, um das Erweiterungsteil (32) des Endoskops (3) dort hindurchzuführen,
**dadurch gekennzeichnet, dass** ein Montageschlitz (15) zwischen dem feststehenden Handstück (11) und dem Betätigungsgriff (12) zum Befestigen des Stücks zur internen Inzision (2) gebildet ist, und
einen Begrenzungsblock an einer Seite des Betätigungsgriffs (12), nahe dem feststehenden Handstück (11) angeordnet ist und aus diesem hervorsteht, um während der Betätigung an einer Seitenwand des feststehenden Handstücks (11) anzuliegen, und eine Nut zum Aufnehmen des Begrenzungsblocks auf dem Betätigungsgriff (12) angeordnet ist, und der Begrenzungsblock drehbar in der Nut angeordnet ist.

2. Multifunktionales endoskopisches System zur Hohlraumuntersuchung und - behandlung nach Anspruch 1, **dadurch gekennzeichnet, dass**
das Arbeitshandstück zur internen Inzision (1) den Typ eines Arbeitshandstücks zur internen Inzision mit kalten Messer annimmt.

3. Multifunktionales endoskopisches System zur Hohlraumuntersuchung und - behandlung nach Anspruch 1, **dadurch gekennzeichnet, dass**
das Arbeitshandstück zur internen Inzision (1) ein Arbeitshandstück zur internen Inzision vom gemischten monopolaren und bipolaren Typ annimmt,
und eine Elektrodenanschlussbuchse (16) am Betätigungsgriff (12) angeordnet ist.

4. Multifunktionales endoskopisches System zur Hohlraumuntersuchung und - behandlung nach Anspruch 1, **dadurch gekennzeichnet, dass**
das Stück zur internen Inzision (2) ein Messer zur internen Inzision umfasst und mit einem Klingenabschnitt (21) an einem vorderen Ende davon und einem Schnappabschnitt (22) an einem hinteren Ende davon gebildet ist; und
eine Vielzahl von zylindrischen Verbindungsstücken (23) an einer äußeren Seitenwand eines Mittelabschnitts des Stücks zur internen Inzision (2) voneinander beabstandet sind, und die Verbindungsstücke (23) an beiden Enden davon offen sind.

5. Multifunktionales endoskopisches System zur Hohlraumuntersuchung und - behandlung nach Anspruch 4, **dadurch gekennzeichnet, dass**
das Messer zur internen Inzision ein kaltes Messer mit langem Griff ist; und das Messer zur internen Inzision einen Messerkopf aufweist, und der Messerkopf gerade, halbrund oder hakenförmig ist.

6. Multifunktionales endoskopisches System zur Hohlraumuntersuchung und - behandlung nach Anspruch 1, **dadurch gekennzeichnet, dass**
das Stück zur internen Inzision (2) eine Elektrode zur internen Inzision umfasst und mit einem Elektrodenabschnitt (24) an einem vorderen Ende des Stücks zur internen Inzision (2) und einem Schnappabschnitt (25) an einem hinteren Ende des Stücks zur internen Inzision (2) gebildet ist; und
ein zylindrisches Verbindungsstück (26) an einer äußeren Seitenwand eines Mittelabschnitts des Stücks zur internen Inzision (2) angeordnet ist, und das Verbindungsstück (23) an beiden Enden davon offen ist.

7. Multifunktionales endoskopisches System zur Hohlraumuntersuchung und - behandlung nach Anspruch 6, **dadurch gekennzeichnet, dass**
der Elektrodenabschnitt (24) der Elektrode zur internen Inzision eine Nadelelektrode oder eine Ringelektrode ist.

8. Multifunktionales endoskopisches System zur Hohlraumuntersuchung und - behandlung nach Anspruch 1, **dadurch gekennzeichnet, dass**
das Endoskop (3) ein gerades, starres Glasfaserendoskoprohr mit einem Durchmesser von weniger als φ2 mm annimmt; und die Endoskophülle (4) einen Durchmesser von weniger als φ2,5 mm aufweist.

## Revendications

1. Système endoscopique multifonctionnel pour une inspection et un traitement de cavité, comprenant
une pièce à main de travail d'incision interne (1) ;
une pièce d'incision interne (2), agencée pour s'encliqueter de manière fixe sur la pièce à main de travail d'incision interne (1),
un endoscope (3), agencé pour être inséré dans la pièce à main de travail d'incision interne (1) à partir d'une extrémité arrière de la pièce à main de travail d'incision interne (1), dans lequel une partie de tête (31) de l'endoscope (3) est encliquetée sur l'extrémité arrière de la pièce à main de travail d'incision interne (1), et une partie d'extension (32) de l'endoscope (3) traverse une cavité interne de la pièce à main de travail d'incision interne (1) et s'étend à partir d'une extrémité avant de la pièce à main de travail d'incision interne (1) ;
une gaine d'endoscope (4), encliquetée sur l'extrémité avant de la pièce à main de travail d'incision interne (1), dans lequel la pièce d'incision interne (2) et la partie d'extension (32) de l'endoscope (3) sont emmanchées axialement dans la gaine d'endoscope (4),
dans lequel la pièce à main de travail d'incision interne (1) comprend :
une pièce à main fixe (11) ;
une poignée d'actionnement (12), agencée sur la pièce à main fixe (11) et utilisée pour pousser la pièce d'incision interne (2) pour qu'elle se déplace vers l'avant et vers l'arrière axialement le long de la partie d'extension (32) de l'endoscope (3), et
une pièce élastique en forme de V (13) pour réinitialiser la poignée d'actionnement (12) ; dans lequel,
un canal (14) est formé entre la pièce à main fixe (11) et la poignée d'actionnement (12) pour faire passer la partie d'extension (32) de l'endoscope (3) à travers celui-ci,
**caractérisé en ce qu'**une fente de montage (15) est formée entre la pièce à main de fixation (11) et la poignée d'actionnement (12) pour fixer la pièce d'incision interne (2), et
un bloc de limitation est agencé sur un côté de la poignée d'actionnement (12) à proximité de la pièce à main fixe (11) et fait saillie à partir de celle-ci, de manière à être contre une paroi latérale de la pièce à main fixe (11) pendant un actionnement, et une rainure pour recevoir le bloc de limitation est agencée sur la poignée d'actionnement (12), et le bloc de limitation est agencé de manière rotative dans la rainure.

2. Système endoscopique multifonctionnel pour une inspection et un traitement de cavité selon la revendication 1, **caractérisé en ce que**
la pièce à main de travail d'incision interne (1) adopte une pièce à main de travail d'incision interne de type couteau à froid.

3. Système endoscopique multifonctionnel pour une inspection et un traitement de cavité selon la revendication 1, **caractérisé en ce que**
la pièce à main de travail d'incision interne (1) adopte une pièce à main de travail d'incision interne de type mixte monopolaire et bipolaire,
et une douille de connexion d'électrode (16) est agencée sur la poignée d'actionnement (12).

4. Système endoscopique multifonctionnel pour une inspection et un traitement de cavité selon la revendication 1, **caractérisé en ce que**
la pièce d'incision interne (2) comprend un couteau d'incision interne et est formée avec une partie de lame (21) au niveau d'une extrémité avant de celle-ci et une partie d'encliquetage (22) au niveau d'une extrémité arrière de celle-ci ; et
une pluralité de pièces de connexion cylindriques (23) sont espacées sur une paroi latérale externe d'une partie médiane de la pièce d'incision interne (2), et la pièce de connexion (23) est ouverte au niveau de ses deux extrémités.

5. Système endoscopique multifonctionnel pour une inspection et un traitement de cavité selon la revendication 4, **caractérisé en ce que**
le couteau d'incision interne est un couteau à froid à long manche ; et le couteau d'incision interne présente une tête de couteau et la tête de couteau est droite, semi-circulaire ou en crochet.

6. Système endoscopique multifonctionnel pour une inspection et un traitement de cavité selon la revendication 1, **caractérisé en ce que**
la pièce d'incision interne (2) comprend une électrode d'incision interne et est formée avec une partie d'électrode (24) au niveau d'une extrémité avant de la pièce d'incision interne (2), et une partie d'encliquetage (25) au niveau d'une extrémité arrière de la pièce d'incision interne (2) ; et
une pièce de connexion cylindrique (26) est agencée sur une paroi latérale externe d'une partie médiane de la pièce d'incision interne (2) et la pièce de connexion (23) est ouverte au niveau de ses deux extrémités.

7. Système endoscopique multifonctionnel pour une inspection et un traitement de cavité selon la revendication 6, **caractérisé en ce que**
la partie électrode (24) de l'électrode d'incision interne est une électrode à aiguille ou une électrode annulaire.

8. Système endoscopique multifonctionnel pour une inspection et un traitement de cavité selon la revendication 1, **caractérisé en ce que**
l'endoscope (3) adopte un endoscope à fibre tubulaire rigide droit d'un diamètre inférieur à φ2 mm ; et la gaine d'endoscope (4) présente un diamètre inférieur à φ2,5 mm.
